Europäisches Patentamt

European Patent Office

Office européen des brevets

(11) Publication number: **0 358 485**
**A2**

# (12) EUROPEAN PATENT APPLICATION

(21) Application number: **89309025.8**

(22) Date of filing: **06.09.89**

(51) Int. Cl.5: **C 07 K 7/04**
**A 61 K 39/125, A 61 K 39/29,**
**A 61 K 39/145,**
**A 61 K 39/135, A 61 K 39/13,**
**A 61 K 39/245,**
**A 61 K 39/205, A 61 K 39/21,**
**A 61 K 39/015, A 61 K 39/295**

(30) Priority: **08.09.88 GB 8821077**

(43) Date of publication of application:
**14.03.90 Bulletin 90/11**

(84) Designated Contracting States:
**BE CH DE FR GB IT LI NL SE**

(71) Applicant: **THE WELLCOME FOUNDATION LIMITED**
**183-193 Euston Road**
**London NW1 2BP (GB)**

(72) Inventor: **Francis, Michael James**
**c/o The Wellcome Foundation Limited Langley Court**
**Beckenham Kent BR3 3BS (GB)**

**Rowlands, David John**
**c/o The Wellcome Foundation Limited Langley Court**
**Beckenham Kent BR3 3BS (GB)**

(74) Representative: **Woods, Geoffrey Corlett et al**
**J.A. KEMP & CO. 14 South Square Gray's Inn**
**London WC1R 5EU (GB)**

(54) Human rhinovirus peptides.

(57) A synthetic peptide comprises:
(i) an epitope of a human rhinovirus (HRV) capable of eliciting T-cell help for production of antibody against an antigen, or
(ii) a modified form of a said epitope wherein an amino acid residue of a said epitope has been replaced by another amino acid residue such that the thus-modified epitope is also capable of eliciting T-cell help; or a pharmaceutically acceptable salt thereof. The synthetic peptide may also comprise a B-cell epitope. Alternatively, a B-cell epitope may be presented by a second peptide.

EP 0 358 485 A2

**Description**

# HUMAN RHINOVIRUS PEPTIDES

This invention relates to synthetic human rhinovirus (HRV) peptides, their preparation and their use as vaccines.

The aims of a good vaccine should be to provide a rapid onset of immunity that is of long duration and provides immunological memory for a subsequent inoculation or encounter with the infectious agent. The vaccine formulation must also be easy to administer, stable, have minimal side effects and produce broad protection in the recipient. These aims are largely met by many existing commercial products. However, conventional vaccines based on inactivated infectious agents present problems. These include the undefined nature of the immunizing antigen, whether the product is truly innocuous, risk associated with handling large amounts of infectious material, stability and limitations on the mode of presentation, generally resulting from problems of stability.

In an attempt to produce more stable and defined vaccines scientists have been studying the immune response to many infectious agents in detail in order to identify the critical epitopes involved in providing protective immunity. Armed with this knowledge it is now possible to mimic such epitopes by producing short peptides and to use these as the basis of a vaccine. The advantages of such peptide based vaccines are numerous. They are chemically defined, stable indefinitely and no infectious material is involved in their manufacture. Furthermore, they can be designed to stimulate the appropriate immune response and provide the opportunity for using novel delivery systems and for targetting the antigen. From the manufacturers viewpoint they should also reduce the need for a large scale production plant and for complex downstream processing of the product.

Despite these clear advantages a number of criticisms have been levelled at peptide based vaccines. These include the requirements for undefined carrier proteins and the belief that the immunogenicity of a peptide antigen could never approach that of the native organism. It was generally assumed that due to their relatively small molecular size many synthetic peptides would behave like haptens and would require coupling to a large "foreign" protein carrier to enhance their immunogenicity. Immunization with such conjugates often resulted in the production of anti-peptide antibodies that totally failed to recognise the native protein or infectious agent due to the method of peptide/carrier linkage. Other problems, of particular relevance to vaccination, that could be encountered were hypersensitivity to the "foreign" carrier protein and poor batch to batch reproducibility of the conjugates.

We have now located helper T-cell epitopes (Th-epitopes) on HRV2. Peptides incorporating these epitopes and the NIm-II sequence of our EP-A-0287395 were capable of eliciting a better antibody response than peptides incorporating the NIm-II sequence alone. Also, the peptides incorporating a Th-epitope were capable of overcoming non-responsiveness and genetic restriction of the response to the peptide which included only the NIm-II sequence. These findings have general applicability.

Accordingly, the present invention provides a synthetic peptide which comprises:

(i) an epitope of a human rhinovirus (HRV) capable of eliciting T-cell help for production of antibody against an antigen, or

(ii) a modified form of a said epitope wherein an amino acid residue of a said epitope has been replaced by another amino acid residue such that the thus-modified epitope is also capable of eliciting T-cell help; or a pharmaceutically acceptable salt thereof.

The synthetic peptide therefore presents an amino acid sequence which is derived from HRV and which is capable of eliciting T-cell help for production of antibody against an antigen. Optionally an amino acid in the sequence may be replaced by another amino acid which does not affect the function of the sequence to elicit T-cell help.

The peptide preferably also presents an amino acid sequence capable of inducing an antibody response to an antigen (a B-cell epitope). Alternatively, the Th-epitope of the invention and the B-cell epitope may be presented as separate peptides, for example in the same delivery vehicle. Such a delivery vehicle incorporating the two epitopes as separate peptides also forms part of the present invention.

The use of the Th-epitope in association with the B-cell epitope can result in a better antibody response to the B-cell epitope than use of the B-cell epitope alone. Peptides comprising the Th-epitope and the B-cell epitope, and delivery vehicles comprising the two epitopes as separate peptides, can therefore be used as vaccines.

The Th-cell epitope must be capable of binding class II major histocompatibility complex (MHC) molecules on the surface of host antigen presenting cells (APC) and B-cells and of subsequently interacting with the T-cell receptor in the form of a trimolecular complex in order to induce B cells to differentiate and proliferate. A number of Th-cell epitopes have been defined from a wide range of proteins and infectious agents. However, no Th-cell epitopes have been defined before on HRV.

Th-cell epitopes on HRV may be identified by a detailed analysis, using in vitro T-cell stimulation techniques of component proteins, protein fragments and peptides to identify appropriate sequences (Goodman and Sercarz, Ann. Rev. Immunol., 1, 465, 1983; Berzofsky, in "The Year in Immunology, Vol. 2" page 151, Karger, Basel, 1986; and Livingstone and Fathman, Ann. Rev. Immunol., 5, 477, 1987). Further, there are now two published algorithms that improve the chances of selecting appropriate peptide sequences with T-cell

EP 0 358 485 A2

stimulating activity from the primary sequence of a protein.

The first algorithm, proposed by DeLisi and Berzofsky (Proc. Natl. Acad. Sci. USA, 82, 7048, 1985), suggests that T cell sites tend to be amphipathic structures i.e. one portion of the molecule is hydrophobic and the other hydrophilic, which are frequently in the form of an alpha-helix. The originators of this hypothesis have published a computer program (Margalit et al, J. Immunol., 138, 2213, 1987) to assist in the identification of amphipathic helices from the primary amino acid sequence of a protein.

The second algorithm, propose by Rothbard (Ann. Inst. Pasteur, 137E, 518, 1986), suggests that each T-cell epitope has within it a sequence composed of a charged residue or glycine followed by two hydrophobic residues and in many cases the next residue will be charged or polar. This algorithm has been refined to consider further residues flanking the two central hydrophobic amino acids and to suggest possible sub-patterns responsible for the genetic restriction of an epitope (Rothbard and Taylor, EMBO J. 7, 93, 1988).

Preferred Th-cell epitopes comprise amino acid residues 35 to 38, 103 to 106 or 251 to 254 from the VP1 capsid protein of HRV2, amino acid residues 27 to 30 from the VP2 capsid protein of HRV2, amino acid residues 59 to 62 from the VP4 capsid protein of HRV2, or equivalent amino acid residues of another HRV. More preferred are HRV2 VP1 amino acid residues 32 to 41, 100 to 109 or 251 to 260, HRV2 VP2 amino acid residues 24 to 33, HRV2 VP4 amino acid residues 56 to 65, and equivalent amino acid residues from other HRVs. In each case amino acids may be replaced by other amino acids which do not affect the function of a sequence, i.e. its ability to elicit T-cell help for a B-cell epitope.

The Th-epitopes derived from HRV2 have the following sequences, using the one letter code:

DAAE (VP1 residues 35 to 38)
EMAQ (VP1 residues 103 to 106)
RALE (VP1 residues 251 to 254)
DVAN (VP2 residues 27 to 30)
DVLE (VP4 residues 59 to 62)
PALDAAETGH (VP1 residues 32 to 41)
NLQEMAQIRR (VP1 residues 100 to 109)
RALEYTRAHR (VP1 residues 251 to 260)
TSQDVANAIV (VP2 residues 24 to 33) and
PVKDVLEKGI (VP4 residues 56 to 65).

The B-cell epitope may be an epitope capable of raising neutralising antibody, for example an epitope of an infectious agent such as a virus, bacterium or protozoan. It may be an epitope of a non-infectious agent such as a growth hormone. As examples of viral epitopes, there may be mentioned hepatitis B virus, influenza virus, foot-and-mouth disease virus, poliovirus, herpes simplex virus, rabies virus, HIV such as HIV-1 or HIV-2 and HRV. A protozoan whose epitopes may be provided in the malaria parasite Plasmodium falciparum.

A preferred B-cell epitope is the HRV NIm-II sequence of our EP-A-0287395. This epitope is defined by amino acid residues 156 to 164 of VP2 of HRV2 or equivalent amino acid residues of another HRV. These amino acids may be replaced by other amino acids which do not affect the antigenicity of the sequence. For HRV2, the NIm-II sequence is
VKAETRLNP.

Equivalent amino acid residues of other HRVs are the VP2 amino acid residues corresponding to the VP2 amino acid residues 156 to 164 of HRV2. In other words, they can be the counterpart VP2 residues of another HRV serotype. These can readily be determined by lining up the VP2 sequence of another HRV with the VP2 sequence of HRV2. This is a straightforward matter because of the homology between VP2 sequences of different types of HRV. In a similar fashion, the equivalent amino acids in other HRV's to the Th-epitopes defined in HRV2 may be deduced.

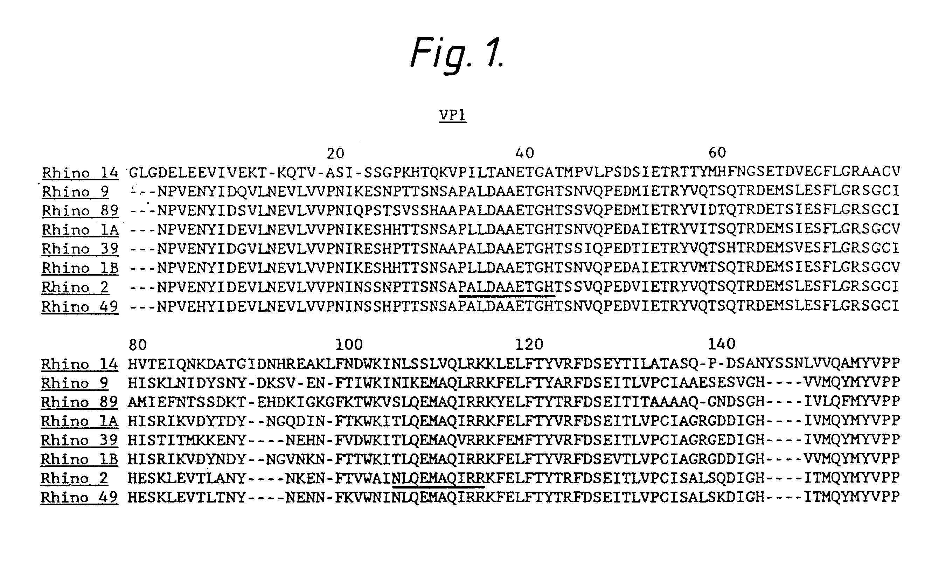

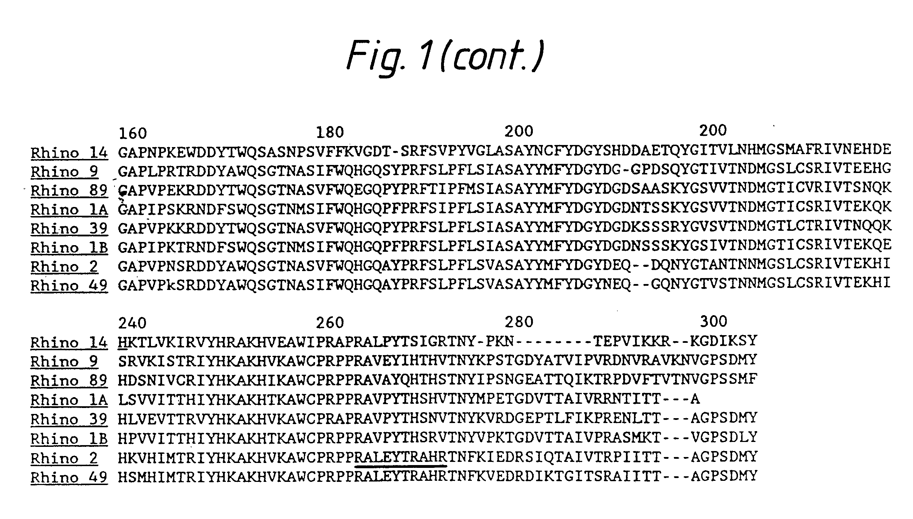

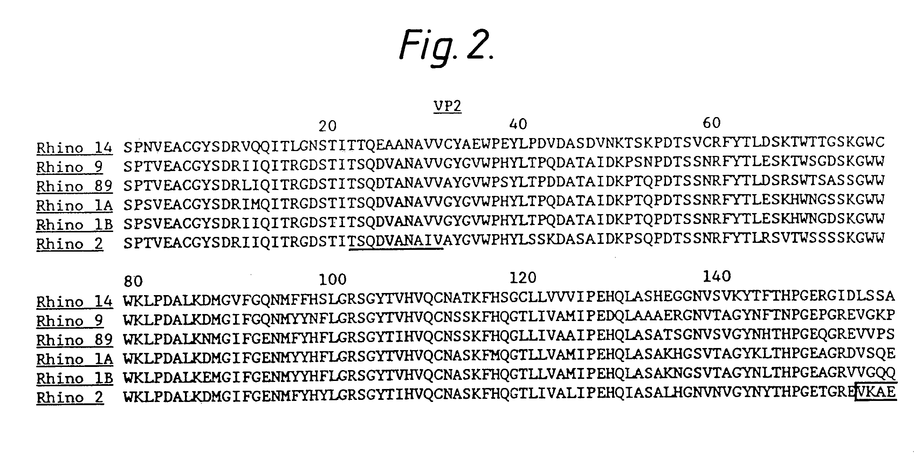

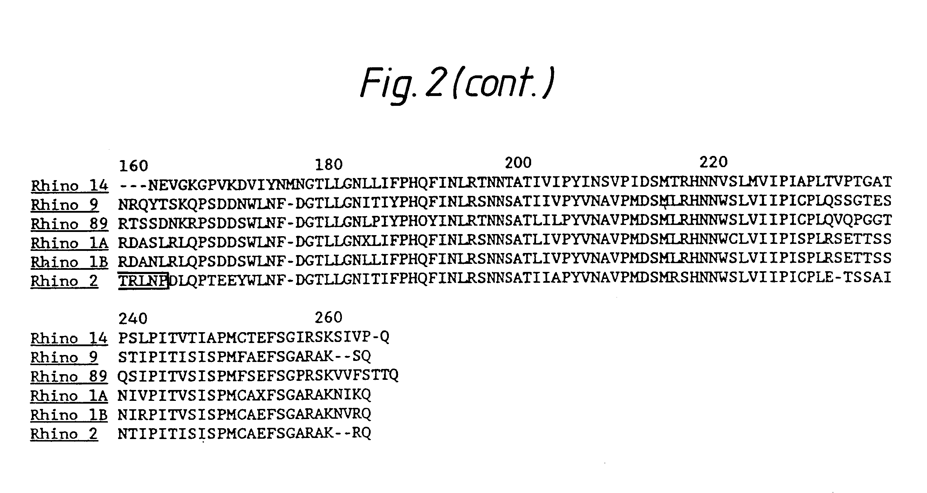

Figures 1 and 2 of the accompanying drawings illustrate this. Here, the VP1 and VP2 sequences of HRV2 and other HRV serotypes have been lined up. The most preferred VP1 and VP2 Th-epitopes have been underlined. The HRV2 NIm-II sequence in VP2 is boxed in Figure 2. For example it can be seen that residues 156 to 161 of HRV14 correspond to residues 156 to 164 of HRV2. These HRV14 residues are:
LSSANE.

One or more amino acids of the defined Th-epitopes and NIm-II sequences may be replaced by one or more other amino acids which do not affect their functionality. Consequently, an amino acid may be replaced by another one which preserves the physicochemical character of the original epitope, i.e. in terms of charge density, hydrophilicity/hydrophobicity, size and configuration, and hence preserve the immunological structure. For example, A may be replaced by G or vice versa; V by A, L or G; K by R; S by T or vice versa; E by D or vice versa; and Q by N or vice versa. Modified amino acids may be provided, for examples esters (such as a $C_1$-$C_4$ alkyl ester) or amides. The carboxy group of the C-terminal amino acid may be thus modified.

The Th-cell epitope and the B-cell epitope are typically present together in the synthetic peptide. The Th-cell epitope may comprise the amino-terminal portion of the peptide and the B-cell epitope may comprise the carboxy-terminal portion of the peptide, or vice versa. The peptide may comprise just the two epitopes. Alternatively, the epitopes may form part of a longer peptide. The epitopes may be linked directly together so that one follows immediately after the other. Alternatively, the epitopes may be separated by intervening spacer amino acid residues.

A longer peptide of up to 50, for example of up to 40 or of up to 30, amino acid residues can be built up. Up to four amino acids may be added to either or both ends of the Th-cell epitope and/or of the B-cell epitope, for

3

example. In one embodiment, it is only to the carboxy-terminus of the NIm-II B-cell epitope sequence that extra amino acid residues are added. Up to 30, for example up to 15 or up to 6, further amino acid residues may be independently provided at either or each terminus of the NIm-II sequence. The Th-cell epitope and the B-cell epitope may be spaced apart by up to 10, for example by up to 6 or up to 3, amino acid residues.

The amino acid residues which may be added to the Th-cell epitope and/or B-cell epitope can be the natural amino acids which occur alongside the epitope in the sequence from which the epitope has been derived. For the NIm-II sequence in HRV2 for example, the amino acid residue at VP2 position 165 is D. Preferably, therefore, this is the first amino acid residue provided at the carboxy-terminus of NIm-II sequence derived from HRV2. Preferred peptides built up in this way from the VP2 sequences of HRV2 and HRV14 respectively are:
VKAETRLNPDLQPTE and
LSSANEVGGPVK.

Further, longer peptides may also comprise more than one Th-cell epitope and/or more than one B-cell epitope. Also, a cysteine (C) residue may be added to either or both terminals of the peptides. In particular, a C residue may be added to the carboxy-terminus alone.

If the Th-epitope and the B-cell epitope are presented as separate peptides, they may also form part of longer peptides. Such longer peptides may have up to 35, for example up to 25 or up to 15, amino acid residues in total. Amino acid residues may be added to either or both ends of either or both epitopes, for example up to four to the N-terminus and/or up to four to the C-terminus. Preferably the additional amino acids are the natural amino acids which occur alongside the Th-epitope or B-cell epitope in the sequence from which the epitope has been derived.

The peptides are synthetic peptides. They may be prepared by chemical synthesis. A peptide may be built up from single amino acids and/or preformed peptides of two or more amino acids in the order of the sequence of the desired peptide. Solid-phase or solution methods may be employed. The resultant peptide may be converted into a pharmaceutically acceptable salt if desired.

In solid-phase synthesis, the amino acid sequence of the desired peptide is built up sequentially from the C-terminal amino acid which is bound to an insoluble resin. When the desired peptide has been produced, it is cleaved from the resin. When solution-phase synthesis is employed, the desired peptide may again be built up from the C-terminal amino acid. The carboxy group of this acid remains blocked throughout by a suitable protecting group, which is removed at the end of the synthesis.

Whichever technique, solid-phase or solution-phase, is employed each amino acid added to the reaction system typically has a protected amino group and an activated carboxy group. Functional side-chain groups are protected too. After each step in the synthesis, the amino-protecting group is removed. Side-chain functional groups are generally removed at the end of the synthesis.

The resultant peptide may then be converted into a pharmaceutically acceptable salt. It may be converted into an acid addition salt with an organic or inorganic acid. Suitable acids include acetic, succinic and hydrochloric acid. Alternatively, the peptide may be converted into a carboxylic acid salt such as the ammonium salt or an alkali metal salt such as the sodium or potassium salt.

The peptides of the invention may also be prepared by recombinant DNA methodologies. Thus, a DNA sequence encoding the peptide is provided. An expression vector is prepared which incorporates the DNA sequence and which is capable of expressing the peptide when provided in a suitable host. The DNA sequence is located between translation start and stop signals in the vector. Appropriate transcriptional control elements are also provided, in particular a promoter for the DNA sequence and a transcriptional termination site. The DNA sequence is provided in the correct frame such as to enable expression of the peptide to occur in a host compatible with the vector.

Any appropriate host-vector system may be employed. The vector may be a plasmid. In that event, a bacterial or yeast host may be used. Alternatively, the vector may be a viral vector. This may be used to transfect cells of a mammalian cell line in order to cause peptide expression.

The peptides of the invention can raise neutralising antibody. They therefore may be used as vaccines for humans. Vaccination is achieved by administering to a patient an effective amount of a peptide. A human may therefore be vaccinated against an antigen by administering thereto an effective amount of the Th-epitope of the invention and a B-cell epitope capable of inducing antibody against the foreign antigen. Preferably a peptide comprising both epitopes is given.

An oral route or a parenteral route such as sub-cutaneously, intravenously or intramuscularly may be adopted. Typically, a peptide is administered in an amount of 1, to 1,000 ug per dose, more preferably 10 to 100 ug per dose, by either the oral or the parenteral route.

For vaccination purposes, a peptide is typically formulated with a pharmaceutically acceptable carrier or diluent. Conventional formulations, carriers, adjuvants and diluents may be employed. These will of course be determined by the route of administration. Suitable carriers and diluents include Freund's incomplete adjuvant (IFA), aluminium hydroxide, saponin, DEAE-dextran, muramyl dipeptide, mineral oils, neutral oils such as miglycol, vegetable oils such as arachis oil, "Iscoms", liposomes, Pluronic (trade mark) polyols or the Ribi adjuvant system (GB-A-2189141). When the Th-epitope and B-cell epitope are presented as separate peptides in the same delivery vehicle, any synthetic particulate delivery system may be used including liposomes, "Iscoms" and oil droplets.

The following Examples illustrate the invention. In the accompanying drawings:
Figure 1 shows the VP1 sequences for HRV2 and other HRV's;

Figure 2 shows the VP2 sequences for HRV2 and other HRV's;
Figure 3 shows the immune response of guinea pigs to peptide 69 with and without a Th-epitope;
Figure 4 shows the immune response of mice to peptide 69 with and without a Th-epitope;
Figure 5 shows the immune response of mice of different haplotypes to peptides 370 and 371; and
Figure 6 shows BALB/c mouse. (H-2$^d$) T-cell proliferation to rhinovirus peptides.

Example 1: Preparation of peptides

The peptides shown below were synthesised by the solid-phase method. More specifically, synthesis was carried out using an adaption of the Merrifield method (Merrifield, JACS, 85, 2149-2154, 1963) described by Houghten (Houghten, Proc. Natl. Acad. Sci. USA, 82, 5131-5135, 1985). Each peptide has an additional non-natural cysteine residue at its C-terminus. For comparative purposes, peptide 69 was synthesised in the same way. Peptide 69 did not incorporate an additional Th-cell epitope.

| PEPTIDE | REFERENCE NUMBER |
|---|---|
| VKAETRLNPDLQPTENLQEMAQIRRC (peptide 69 + HRV2 VP1 100-109 + C) | 370 |
| VKAETRLNPDLQPTERALEYTRAHRC (peptide 69 + HRV2 VP1 251-260 + C) | 371 |
| VKAETRLNPDLQPTETSQDVANAIVC (peptide 69 + HRV2 VP2 24-33 + C) | 525 |
| VKAETRLNPDLQPTEPALDAAETGHC (peptide 69 + HRV2 VP1 32-41 + C) | 526 |
| VKAETRLNPDLQPTEPVKDVLEKGIC (peptide 69 + HRV2 VP4 56-65 + C) | 527 |
| VKAETRLNPDLQPTEC (comparison; HRV2 VP2 156-170 + C) | 69 |

Example 2: Tests of the peptides

(a) Immunogenicity in guinea pigs

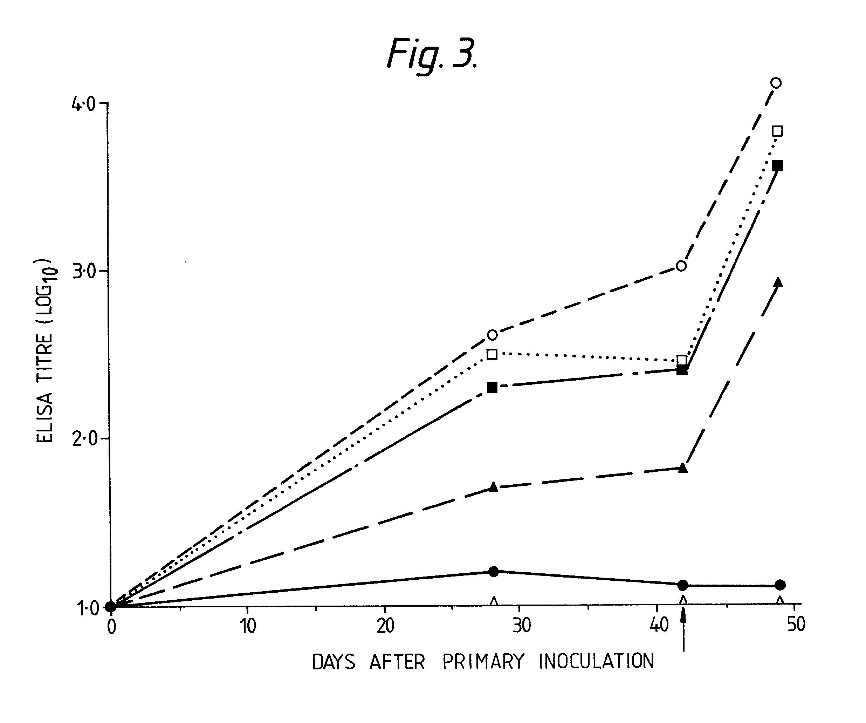

Six groups of between four and eight guinea pigs each were inoculated intramuscularly with 200 ug of either peptide 69 or one of peptides 370, 371 and 525 to 527. The peptides were administered with IFA. These animals were boosted at day 42 (arrow in Figure 3).

Results of anti-peptide 69 activity as measured by indirect ELISA in sera collected at 0, 28, 42 and 49 days are given in Figure 3. In Figure 3, peptide 69 is denoted by black circles; peptide 370 by black squares; peptide 371 by black triangles; Peptide 525 by open circles; peptide 526 by open squares and peptide 527 by open triangles.

It can be seen that peptide 69 above and peptide 527 (peptide sequence 69 + T cell site VP4 56-65 + C) failed to elicit a primary or secondary response. In contrast peptides 370, 371, 525 and 526, which contained the peptide 69 sequence plus four different T cell sites, all produced a good primary and secondary response following inoculation. Therefore, the inclusion of these T cell sites within the peptide had overcome the non-responsiveness of guinea pigs to peptide 69 alone.

(b) Immunogenicity in mice

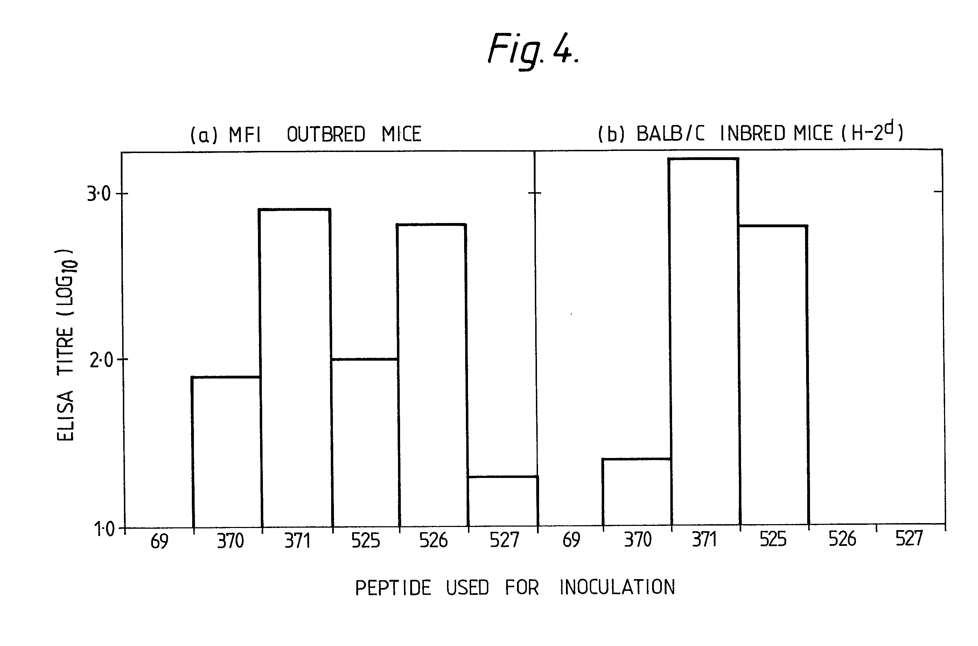

Seven groups each of between eight and sixteen MF1 mice and seven groups each of eight BALB/c mice were inoculated with 200 ug of either peptide 69 alone or one of peptides 370, 371 and 525 to 527, plus IFA. Results of anti-peptide 69 activity as measured by indirect ELISA in sera collected at 28 days after inoculation are given in Figure 4. MF1 mice failed to respond to peptide 69 alone. However, each of the peptides containing the peptide 69 sequence plus a Th-epitope produced an anti-peptide 69 response (Fig. 4a). Of these peptides 371 and 525 were particularly immunogenic in BALB/c mice (Fig. 4b).

5

## EP 0 358 485 A2

(c) Genetic restriction of peptides 370 and 371 in congenic mice

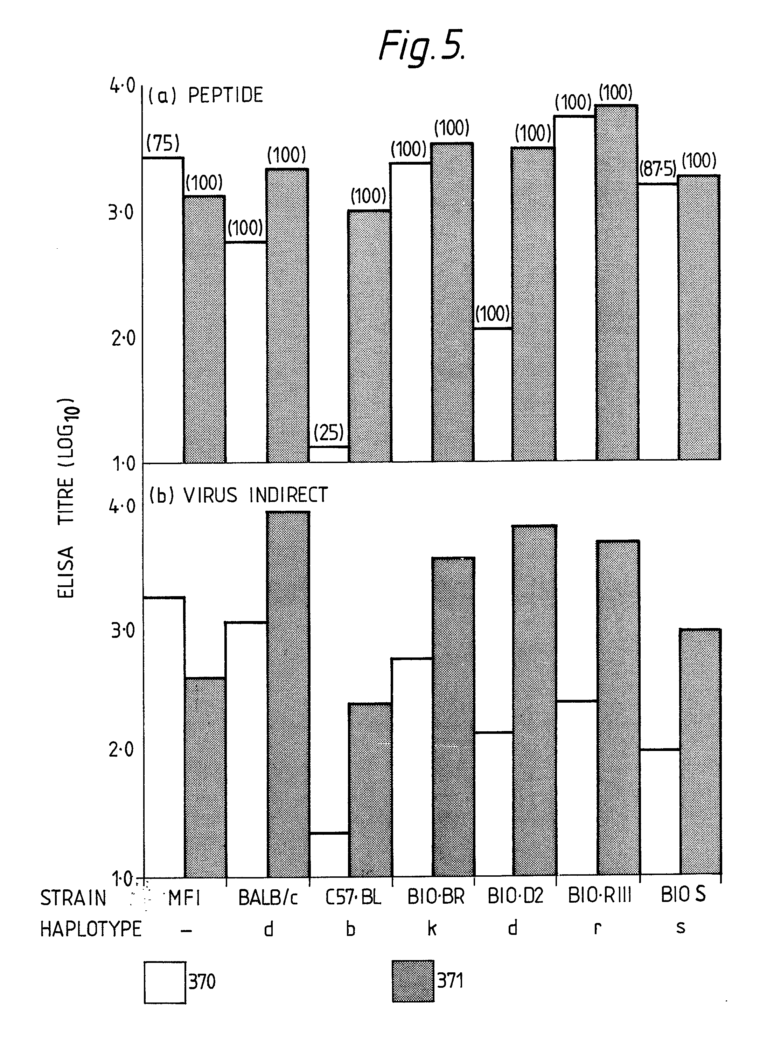

Seven groups each of 8 different haplotype mice were immunized on day 0 with 200 ug of peptides 370 and 371 in IFA. Serum samples collected at 84 days were tested for anti-peptide 69 and anti-virus activity by indirect ELISA. The results of anti-peptide 69 ELISA tests are shown in Figure 5a. Percent responder animals are given in parenthesis.

Figure 5a shows differences in the immunogenicity of 370 and 371 in the different mouse strains. C57BR10 (H-2$^b$) mice appeared to be a poor responder strain to peptide 370. Overall, fewer mice immunized with peptide 370 were responders when compared to 371. However, those strains which were poor primary responders to 370 eventually raise anti-peptide 69 antibodies following a boost. Peptide 371 appeared to be highly immunogenic in all mouse strains tested.

This broad cross reactivity is a desirable property of any T-cell sites incorporated within a peptide vaccine. Furthermore, most of the antibodies raised to 371 appear to be directed to the peptide 69 region. The reactivity of this sera against HRV2 (Fig. 5b) showed similar responses to the anti-peptide 69.

Example 3: Location of Th-cell site in relation to B cell site

In order to examine the importance of the Th-cell site in relation to the B-cell site it is regulating, three peptides were synthesised with VP1 residues 251-260 and peptide 69 in different locations. The peptides were synthesised as described in Example 1. The sequences of these peptides were as follows:

ref: 559

VKAETRLNPDLQPTEGGGGGGRALEYTRAHRC

(Peptide 69 + 5x(gly) + HRV2 VP1 251-260 + Cys)

ref: 560

RALEYTRAHRVKAETRLNPDLQPTEC

(HRV2 VP1 251-260 + peptide 69 + Cys)

ref: 561 (New synthesis of no 371)

VKAETRLNPDLQPTERALEYTRAHRC

(peptide 69 + HRV2 VP1 251-260 + Cys)

A 200 µg dose of each peptide in incomplete Freund's adjuvant was inoculated intramuscularly into groups of four guinea pigs. All animals were boosted with the same preparation 42 days later. Sera collected at 0, 28, 42, 56 and 70 days were analysed for antibody against peptide 69 using an indirect ELISA. Results are given below:

| Peptide No. | Days post primary inoculation | | | | |
|---|---|---|---|---|---|
| | 0 | 28 | 42 | 56 | 70 |
| 559 | <1* | 1.6 | 1.5 | 4.1 | 3.9 |
| 560 | <1 | 1.5 | 1.8 | 2.9 | 2.9 |
| 561 | <1 | 1.3 | 1.3 | 3.1 | 2.8 |

* $\log_{10}$ ELISA endpoint titre

These results indicate that all of the B/Th-cell epitope combinations tested were immunogenic while peptide 69 alone would be non-immunogenic under the same conditions. Furthermore the presence of five glycine residues between the B and Th-cell peptides may, in this case, actually improve the immunogenicity.

Example 4: In vitro proliferative response to rhinovirus peptides

Groups of inbred BALB/c mice were immunized with 6 rhinovirus peptides composed of peptide 69 sequence (B3) plus one of 5 possible Th-cell epitopes (T1-T5) or 10 extra amino acid residues of natural HRV2 VP2 sequence. Peptide numbers were as follows:

| Peptide no. | Sequence |
|---|---|
| 526 (Example 1) | B3 + T1 + cys |
| 370 (Example 1) | B3 + T2 + cys |
| 371 (Example 1) | B3 + T3 + cys |
| 525 (Example 1) | B3 + T4 + cys |
| 527 (Example 1) | B3 + T5 + cys |
| 528 | B3 + 10 extra VP2 residues + cys |

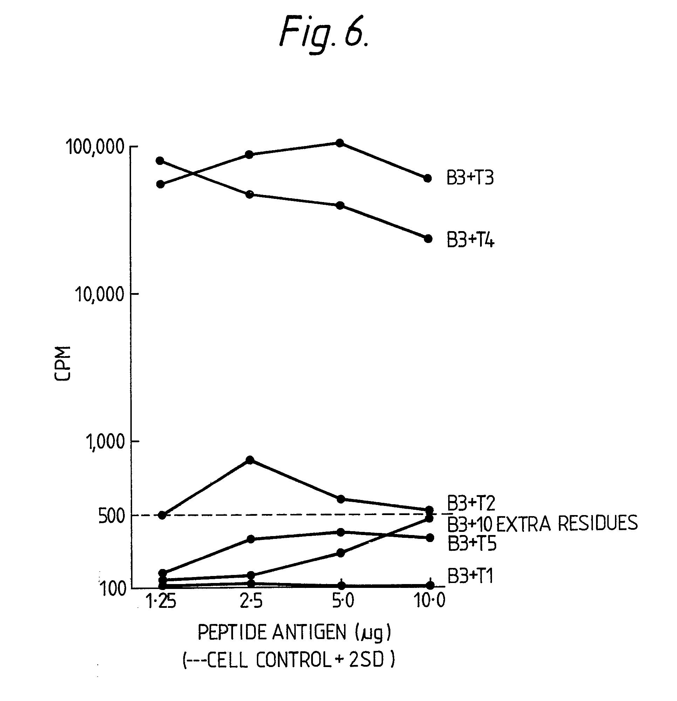

At 7 days post immunization mice were sacrificed and draining inguinal and para-aortic lymph nodes were removed. Single cell suspensions were prepared and incubated at 37°C with each of the immunizing peptides for 3 to 4 days. Radiolabelled thymidine was then added to the cultures and they were assessed for thymidine uptake after 18 hours. The level of thymidine incorporation by the cells, measured in counts per minute (CPM), was taken as an indicator of T-cell proliferative activity in responsse to each peptide. Results, given in Figure 6, indicate that peptides B3 + T3 (371) and B3 + T4 (525) had the strongest T-cell stimulatory activity. These

6

EP 0 358 485 A2

two peptides were the most immunogenic in vivo in BALB/c mice (Figure 4). Peptide B3 + T2 (370) also produced proliferative activity above that of the cell control and this peptide elicited a low but significant primary antibody response in BALB/c mice. Therefore, each peptide that has been shown to be immunogenic in vivo also contains a sequence that will stimulate in vitro T-cell proliferation.

The amino acid composition of peptides 370, 371, 525, 526 and 527 was determined. Amino acid analysis was according to the method of Simpson et al, J. Biol. Chem. 251, 1936-1940, 1976. The results are shown below.

Amino acid analysis of peptide 370

| Amino acid | Observed | Actual |
|---|---|---|
| D+N | 2.87(3) | 3 |
| T | 1.65(2) | 2 |
| E+Q | 6.32(6) | 6 |
| A | 1.90(2) | 2 |
| V | 0.83(1) | 1 |
| M | 1.12(1) | 1 |
| I | 1.14(1) | 1 |
| L | 2.79(3) | 3 |
| K | 0.73(1) | 1 |
| R | 3.40(3) | 3 |
| P | 2.27(2) | 2 |
| C | 1.05(1) | 1 |
| I | 0.95(1) | 1 |

Amino acid analysis of peptide 371

| Amino acid | Observed | Actual |
|---|---|---|
| D+N | 1.91(2) | 2 |
| T | 2.78(3) | 3 |
| E+Q | 3.98(4) | 4 |
| A | 3.03(3) | 3 |
| V | 0.74(1) | 1 |
| L | 2.86(3) | 3 |
| Y | 1.18(1) | 1 |
| H | 1.26(1) | 1 |
| K | 0.69(1) | 1 |
| R | 4.47(4) | 4 |
| P | 2.08(2) | 2 |
| C | 0.97(1) | 1 |
| H | 1.03(1) | 1 |

7

Amino acid analysis of peptide 525

| Amino acid | Observed | Actual |
|---|---|---|
| D+N | 4.07(4) | 4 |
| T | 2.90(3) | 3 |
| S | 1.30(1) | 1 |
| E+Q | 3.97(4) | 4 |
| A | 3.25(3) | 3 |
| V | 2.93(3) | 3 |
| I | 0.87(1) | 1 |
| L | 1.86(2) | 2 |
| K | 0.78(1) | 1 |
| R | 0.76(1) | 1 |
| P | 2.29(2) | 2 |
| C | 1.24(1) | 1 |
| I | 0.95(1) | 1 |
| K | 0.86(1) | 1 |
| R | 0.96(1) | 1 |

Amino acid analysis of peptide 526

| Amino acid | Observed | Actual |
|---|---|---|
| D+N | 2.66(3) | 3 |
| T | 2.86(3) | 3 |
| E+Q | 4.05(4) | 4 |
| G | 1.11(1) | 1 |
| A | 4.19(4) | 4 |
| V | 0.86(1) | 1 |
| L | 2.99(3) | 3 |
| H | 1.14(1) | 1 |
| K | 0.85(1) | 1 |
| R | 0.91(1) | 1 |
| P | 3.41(3) | 3 |
| C | 1.00(1) | 1 |
| G | 1.06(1) | 1 |
| R | 0.95(1) | 1 |

Amino acid analysis of peptide 527

| Amino acid | Observed | Actual |
|---|---|---|
| D+N | 2.49(2) | 3 |
| T | 1.82(2) | 2 |
| E+Q | 4.03(4) | 4 |
| G | 1.21(1) | 1 |
| A | 0.92(1) | 1 |
| V | 3.01(3) | 3 |
| I | 0.95(1) | 1 |
| L | 3.12(3) | 3 |
| K | 3.10(3) | 3 |
| R | 0.96(1) | 1 |
| P | 3.37(3) | 3 |
| C | 1.00(1) | 1 |
| G | 1.21(1) | 1 |
| A | 0.75(1) | 1 |
| I | 1.08(1) | 1 |
| R | 0.96(1) | 1 |

## Claims

1. A synthetic peptide which comprises:

(i) an epitope of a human rhinovirus (HRV) capable of eliciting T-cell help for production of antibody against an antigen, or

(ii) a modified form of a said epitope wherein an amino acid residue of a said epitope has been replaced by another amino acid residue such that the thus-modified epitope is also capable of eliciting T-cell help; or a pharmaceutically acceptable salt thereof.

2. A synthetic peptide or salt thereof according to claim 1, wherein the said epitope comprises amino acid residues 35 to 38, 103 to 106 or 251 to 254 from the VP1 capsid protein of HRV2, amino acid residues 27 to 30 from the VP2 capsid protein of HRV2 or amino acid residues 59 to 62 from the VP4 capsid protein of HRV2; or equivalent amino acid residues of another HRV.

3. A synthetic peptide or salt thereof according to claim 1, wherein the said epitope comprises HRV2 VP1 residues 32 to 41, 100 to 109 or 251 to 260, HRV2 VP2 amino acid residues 24 to 33 or HRV2 VP2 residues 56 to 65; or equivalent amino acid residues of another HRV.

4. A synthetic peptide or salt thereof according to any one of the preceding claims, which further comprises an amino acid sequence capable of inducing said antibody.

5. A synthetic peptide or salt thereof according to claim 4, wherein the said amino acid sequence is an epitope of a virus, bacterium or protozoan or of a growth hormone.

6. A synthetic peptide or salt thereof according to claim 5, wherein the epitope is an epitope of hepatitis B virus, influenza virus, foot-and-mouth disease virus, poliovirus, herpes simplex virus, rabies virus, HIV-1, HRV or Plasmodium falciparum.

7. A synthetic peptide or salt thereof according to any one of the preceding claims, wherein the synthetic peptide consists of up to 40 amino acid residues.

8. A process for the preparation of a synthetic peptide or salt thereof as defined in any one of the preceding claims, which process comprises preparing the said peptide by chemical synthesis or by recombinant DNA methodology and, if desired, converting the resulting peptide into a pharmaceutically acceptable salt thereof.

9. A vaccine comprising a synthetic peptide or salt thereof as defined in any one of the preceding claims and a pharmaceutically acceptable carrier or diluent.

10. A vaccine according to claim 9, comprising a first synthetic peptide or salt thereof as defined in any one of claims 1 to 3 and a second synthetic peptide, or a pharmaceutically acceptable salt thereof, which comprises an amino acid sequence capable of inducing said antibody.

9

# Fig. 1.

<u>VP1</u>

```
                        20                      40                      60
Rhino 14  GLGDELEEVIVEKT-KQTV-ASI-SSGPKHTQKVPILTANETGATMPVLPSDSIETRTTYMHFNGSETDVECFLGRAACV
Rhino 9   ---NPVENYIDQVLNEVLVVPNIKESNPTTSNSAPALDAAETGHTSNVQPEDMIETRYVQTSQTRDEMSLESFLGRSGCI
Rhino 89  ---NPVENYIDSVLNEVLVVPNIQPSTSVSSHAAPALDAAETGHTSSVQPEDMIETRYVIDTQTRDETSIESFLGRSGCI
Rhino 1A  ---NPVENYIDEVLNEVLVVPNIKESHHTTSNSAPLLDAAETGHTSNVQPEDAIETRYVITSQTRDEMSIESFLGRSGCV
Rhino 39  ---NPVENYIDGVLNEVLVVPNIRESHPTTSNAAPALDAAETGHTSSIQPEDTIETRYVQTSHTRDEMSVESFLGRSGCI
Rhino 1B  ---NPVENYIDEVLNEVLVVPNIKESHHTTSNSAPLLDAAETGHTSNVQPEDAIETRYVMTSQTRDEMSIESFLGRSGCV
Rhino 2   ---NPVENYIDEVLNEVLVVPNINSSNPTTSNSAPALDAAETGHTSSVQPEDVIETRYVQTSQTRDEMSLESFLGRSGCI
Rhino 49  ---NPVEHYIDEVLNEVLVVPNINSSHPTTSNSAPALDAAETGHTSNVQPEDVIETRYVQTSQTRDEMSLESFLGRSGCI


          80                      100                     120                     140
Rhino 14  HVTEIQNKDATGIDNHREAKLFNDWKINLSSLVQLRKKLELFTYVRFDSEYTILATASQ-P-DSANYSSNLVVQAMYVPP
Rhino 9   HISKLNIDYSNY-DKSV-EN-FTIWKINIKEMAQLRRKFELFTYARFDSEITLVPCIAAESESVGH----VVMQYMYVPP
Rhino 89  AMIEFNTSSDKT-EHDKIGKGFKTWKVSLQEMAQIRRKYELFTYRFDSEITITAAAAQ-GNDSGH----IVLQFMYVPP
Rhino 1A  HISRIKVDYTDY--NGQDIN-FTKWKITLQEMAQIRRKFELFTYVRFDSEITLVPCIAGRGDDIGH----IVMQYMYVPP
Rhino 39  HISTITMKKENY----NEHN-FVDWKITLQEMAQVRRKFEMFTYVRFDSEITLVPCIAGRGEDIGH----IVMQYMYVPP
Rhino 1B  HISRIKVDYNDY--NGVNKN-FTTWKITLQEMAQIRRKFELFTYVRFDSEVTLVPCIAGRGDDIGH----VVMQYMYVPP
Rhino 2   HESKLEVTLANY----NKEN-FTVWAINLQEMAQIRRKFELFTYRFDSEITLVPCISALSQDIGH----ITMQYMYVPP
Rhino 49  HESKLEVTLTNY----NENN-FKVWNINLQEMAQIRRKFELFTYTRFDSEITLVPCISALSKDIGH----ITMQYMYVPP
```

EP 0 358 485 A2

# Fig. 1 (cont.)

```
              160                 180                 200                 200
Rhino 14  GAPNPKEWDDYTWQSASNPSVFFKVGDT-SRFSVPYVGLASAYNCFYDGYSHDDAETQYGITVLNHMGSMAFRIVNEHDE
Rhino  9  GAPLPRTRDDYAWQSGTNASIFWQHGQSYPRFSLPFLSIASAYYMFYDGYDG-GPDSQYGTIVTNDMGSLCSRIVTEEHG
Rhino 89  GAPVPEKRDDYTWQSGTNASVFWQEGQPYPRFTIPFMSIASAYYMFYDGYDGDSAASKYGSVVTNDMGTICVRIVTSNQK
Rhino 1A  GAPIPSKRNDFSWQSGTNMSIFWQHGQPFPRFSIPFLSIASAYYMFYDGYDGDNTSSKYGSVVTNDMGTICSRIVTEKQK
Rhino 39  GAPVPKKRDDYTWQSGTNASVFWQHGQPYPRFSLPFLSIASAYYMFYDGYDGDKSSSRYGVSVTNDMGTLCTRIVTNQQK
Rhino 1B  GAPIPKTRNDFSWQSGTNMSIFWQHGQPFPRFSLPFLSIASAYYMFYDGYDGDNSSSKYGSIVTNDMGTICSRIVTEKQE
Rhino  2  GAPVPNSRDDYAWQSGTNASVFWQHGQAYPRFSLPFLSVASAYYMFYDGYDEQ--DQNYGTANTNNMGSLCSRIVTEKHI
Rhino 49  GAPVPkSRDDYAWQSGTNASIFWQHGQAYPRFSLPFLSVASAYYMFYDGYNEQ--GQNYGTVSTNNMGSLCSRIVTEKHI


              240                 260                 280                 300
Rhino 14  HKTLVKIRVYHRAKHVEAWIPRAPRALPYTSIGRTNY-PKN--------TEPVIKKR--KGDIKSY
Rhino  9  SRVKISTRIYHKAKHVKAWCPRPPRAVEYIHTHVTNYKPSTGDYATVIPVRDNVRAVKNVGPSDMY
Rhino 89  HDSNIVCRIYHKAKHIKAWCPRPPRAVAYQHTHSTNYIPSNGEATTQIKTRPDVFTVTNVGPSSMF
Rhino 1A  LSVVITTHIYHKAKHTKAWCPRPPRAVPYTHSHVTNYMPETGDVTTAIVRRNTITT---A
Rhino 39  HLVEVTTRVYHKAKHVKAWCPRAPRAVPYTHSNVTNYKVRDGEPTLFIKPRENLTT---AGPSDMY
Rhino 1B  HPVVITTHIYHKAKHTKAWCPRPPRAVPYTHSRVTNYVPKTGDVTTAIVPRASMKT---VGPSDLY
Rhino  2  HKVHIMTRIYHKAKHVKAWCPRPPRALEYTRAHRTNFKIEDRSIQTAIVTRPIITT---AGPSDMY
Rhino 49  HSMHIMTRIYHKAKHVKAWCPRPPRALEYTRAHRTNFKVEDRDIKTGITSRAIITT---AGPSDMY
```

EP 0 358 485 A2

## Fig. 2.

EP 0 358 485 A2

VP2

          20                      40                      60

Rhino 14  SPNVEACGYSDRVQQITLGNSTITTQEAANAVVCYAEWPEYLPDVDASDVNKTSKPDTSVCRFYTLDSKTWTTGSKGWC

Rhino 9   SPTVEACGYSDRIIQITRGDSTITSQDVANAVVGYGVWPHYLTPQDATAIDKPSNPDTSSNRFYTLESKTWSGDSKGWW

Rhino 89  SPTVEACGYSDRLIQITRGDSTITSQDTANAVVAYGVWPSYLTPDDATAIDKPTQPDTSSNRFYTLDSRSWTSASSGWW

Rhino 1A  SPSVEACGYSDRIMQITRGDSTITSQDVANAVVGYGVWPHYLTPQDATAIDKPTQPDTSSNRFYTLESKHWNGSSKGWW

Rhino 1B  SPSVEACGYSDRIIQITRGDSTITSQDVANAVVGYGVWPHYLTPQDATAIDKPTQPDTSSNRFYTLESKHWNGDSKGWW

Rhino 2   SPTVEACGYSDRIIQITRGDSTITSQDVANAIVAYGVWPHYLSSKDASAIDKPSQPDTSSNRFYTLRSVTWSSSSKGWW


          80                  100                  120                  140

Rhino 14  WKLPDALKDMGVFGQNMFFHSLGRSGYTVHVQCNATKFHSGCLLVVVIPEHQLASHEGGNVSVKYTFTHPGERGIDLSSA

Rhino 9   WKLPDALKDMGIFGQNMYYNFLGRSGYTVHVQCNSSKFHQGTLIVAMIPEDQLAAAERGNVTAGYNFTNPGEPGREVGKP

Rhino 89  WKLPDALKNMGIFGENMFYHFLGRSGYTIHVQCNSSKFHQGLLIVAAIPEHQLASATSGNVSVGYNHTHPGEQGREVVPS

Rhino 1A  WKLPDALKDMGIFGENMYYHFLGRSGYTVHVQCNASKFMQGTLLVAMIPEHQLASAKHGSVTAGYKLTHPGEAGRDVSQE

Rhino 1B  WKLPDALKEMGIFGENMYYHFLGRSGYTVHVQCNASKFHQGTLLVAMIPEHQLASAKNGSVTAGYNLTHPGEAGRVVGQQ

Rhino 2   WKLPDALKDMGIFGENMFYHYLGRSGYTIHVQCNASKFHQGTLIVALIPEHQIASALHGNVNVGYNYTHPGETGREVKAE

## Fig. 2 (cont.)

```
              160                180                200                220
Rhino 14  ---NEVGKGPVKDVIYNMNGTLLGNLLIFPHQFINLRTNNTATIVIPYINSVPIDSMTRHNNVSLMVIPIAPLTVPTGAT
Rhino 9   NRQYTSKQPSDDNWLNF-DGTLLGNITIYPHQFINLRSNNSATIIVPYVNAVPMDSMLRHNNWSLVIIPICPLQSSGTES
Rhino 89  RTSSDNKRPSDDSWLNF-DGTLLGNLPIYPHOYINLRTNNSATLILPYVNAVPMDSMLRHNNWSLVIIPICPLQVQPGGT
Rhino 1A  RDASLRLQPSDDSWLNF-DGTLLGNXLIFPHQFINLRSNNSATLIVPYVNAVPMDSMLRHNNWCLVIIPISPLRSETTSS
Rhino 1B  RDANLRLQPSDDSWLNF-DGTLLGNLLIFPHQFINLRSNNSATLIVPYVNAVPMDSMLRHNNWSLVIIPISPLRSETTSS
Rhino 2   TRLNFDLQPTEEYWLNF-DGTLLGNITIFPHQFINLRSNNSATIIAPYVNAVPMDSMRSHNNWSLVIIPICPLE-TSSAI

              240                260
Rhino 14  PSLPITVTIAPMCTEFSGIRSKSIVP-Q
Rhino 9   STIPITISISPMFAEFSGARAK--SQ
Rhino 89  QSIPITVSISPMFSEFSGPRSKVVFSTTQ
Rhino 1A  NIVPITVSISPMCAXFSGARAKNIKQ
Rhino 1B  NIRPITVSISPMCAEFSGARAKNVRQ
Rhino 2   NTIPITISISPMCAEFSGARAK--RQ
```

EP 0 358 485 A2

Fig. 3.

ELISA TITRE (LOG₁₀)

4·0

3·0

2·0

1·0

0    10    20    30    40    50

DAYS AFTER PRIMARY INOCULATION

Fig. 4.

(a) MFI OUTBRED MICE      (b) BALB/C INBRED MICE (H-2$^d$)

ELISA TITRE (LOG$_{10}$)

PEPTIDE USED FOR INOCULATION

EP 0 358 485 A2

# Fig.5.

(a) PEPTIDE

ELISA TITRE (LOG_10)

(b) VIRUS INDIRECT

| STRAIN | MFI | BALB/c | C57·BL | BIO·BR | BIO·D2 | BIO·RIII | BIO S |
|--------|-----|--------|--------|--------|--------|----------|-------|
| HAPLOTYPE | — | d | b | k | d | r | s |

□ 370    ▨ 371

# Fig. 6.

PEPTIDE ANTIGEN (μg)
(---CELL CONTROL + 2SD )